# EUROPÄISCHE PATENTSCHRIFT

(11) **EP 1 159 064 B1**
(45) Veröffentlichungstag und Bekanntmachung des Hinweises auf die Patenterteilung: **28.04.2004**
(21) Anmeldenummer: 00912603.8
(22) Anmeldetag: 13.03.2000
(51) Int. Cl.: B01J 19/00, C07H 21/00

(54) **VERFAHREN ZUR PHOTOLYTISCHEN ABSPALTUNG VON SCHUTZGRUPPEN VON IMMOBILISIERTEN NUCLEOSID-DERIVATEN, INSBESONDERE IN DER DNA-CHIP-HERSTELLUNG**
METHOD FOR PHOTOLYTICALLY DEPROTECTING IMMOBILIZED NUCLEOSIDE DERIVATIVES, ESPECIALLY IN THE PRODUCTION OF DNA CHIPS
TECHNIQUE DE SEPARATION PHOTOLYTIQUE DES GROUPES PROTECTEURS DE DERIVES NUCLEOSIDIQUES IMMOBILISES, NOTAMMENT POUR LA FABRICATION DES PUCES A ADN

(30) Priorität: 11.03.1999 DE 19910808; 05.11.1999 DE 19953289
(43) Veröffentlichungstag der Anmeldung: 05.12.2001
(73) Patentinhaber: NIGU CHEMIE GMBH, D-84478 Waldkraiburg (DE); Stengele, Klaus-Peter, Dr., 84568 Pleiskirchen (DE)
(72) Erfinder: STENGELE, Klaus-Peter, D-84568 Pleiskirchen (DE); GIEGRICH, Heinrich, D-84478 Waldkraiburg (DE)
(74) Vertreter: Stein-Dräger, Christiane
(86) Internationale Anmeldenummer: PCT/EP2000/002197
(87) Internationale Veröffentlichungsnummer: WO 2000/053309

(56) Entgegenhaltungen:
- WO-A-98/39348
- US-A- 5 658 734
- MCGALL G. H. ET AL.: "The efficiency of light-directed synthesis of DNA arrays on glass substrates" J. AM. CHEM. SOC., Bd. 119, Nr. 22, 1997, Seiten 5080-5090, XP000775689 in der Anmeldung erwähnt

## Beschreibung

Die vorliegende Erfindung betrifft ein Verfahren zur gezielten photolytischen Schutzgruppen-Abspaltung von immobilisierten Nucleosid-Derivaten, insbesondere in der photolithographischen Herstellung von DNA-Chips.

Die Herstellung von DNA-Chips für die Analytik und Diagnostik in der Molekularbiologie, Medizin und verwandten Gebieten erfolgt aus Gründen der Parallelisierung und Miniaturisierung üblicherweise mit photolithographischen Techniken. Hierbei werden die Nucleosid-Derivate mit photolabilen Schutzgruppen auf geeigneten Trägermaterialien immobilisiert. Im Anschluß daran erfolgt dann die gezielte Schutzgruppen-Abspaltung durch Photolyse. Aufgrund fehlender postsynthetischer Reinigungsmethoden werden an die Chemie der Schutzgruppen sehr hohe Anforderungen gestellt.

Entsprechend dem Stand der Technik gibt es für die Durchführung der photolytischen Schutzgruppen-Abspaltung zwei Verfahren. Beim ersten Verfahren erfolgt die Belichtung der DNA-Chips in einer Durchflußkammer mit einem geeigneten Lösemittel oder Lösemittelgemisch (vgl. G.H. McGall, A.D. Barone, M. Diggelmann, S.P.A. Fodor, E. Gentalen, N. Ngo, J. Am. Chem. Soc. 1997, 119, 5081-5090). Hierbei wird das Trägermaterial (bspw. in Form eines Glasträgers) mit den immobilisierten Nucleosid-Derivaten in eine Durchflußkammer eingebaut. Während der Bestrahlung wird eine geeignete Lösemittelmischung durch die Durchflußkammer gepumpt, wodurch die Syntheseseite des Substrats benetzt wird und die immobilisierten wachsenden DNA-Ketten quasi in Lösung vorliegen. Somit ist eine Beteiligung des Lösemittels oder Lösemittelgemisches während der lichtgesteuerten Schutzgruppenabspaltung auf jeden Fall gewährleistet. Konstruktionsbedingt erfolgt die Belichtung der Chipoberfläche von der "falschen" Seite, d. h. von hinten durch das Trägermaterial (bspw. in Form von Glasplättchen).

Diese Verfahrensmethode ist mit einigen Nachteilen behaftet. So bedingt die Lichtstreuung am Glasträger eine schlechte optische Auflösung. Außerdem kann es durch die Erwärmung des Trägermaterials sowie eine nicht ausreichende Benetzung der Trägermaterialoberfläche zu thermischen und photolytischen Nebenreaktionen kommen. Da die abzuspaltende photolabile Schutzgruppe quasi am anderen Ende des Lichtweges liegt, kann die ihr vorliegende Oligonucleotid-Kette als Lichtfilter fungieren, was zum einen die Gefahr von photolytischen Nebenreaktionen in sich birgt und zum anderen eine Verlängerung der Belichtungszeit verursachen kann.

Beim zweiten bekannten Verfahren zur photolithographischen Herstellung von DNA-Chips werden diese ohne Zuhilfenahme eines Lösemittels von der "richtigen" Seite, d. h. von vorne, belichtet (vgl. M.C. Pirrung, L. Fallon, G. McGall, J. Org. Chem. 1998, 63, 241-246 sowie die WO-A-98 39 348). Als besonders nachteilig bei diesem Verfahren hat sich die schlechte Qualität der synthetisierten Oligonucleotide erwiesen, was auf eine langsame und unvollständige Entschützung der Nucleosid-Derivate sowie auf thermische bzw. photolytische Nebenreaktionen zurückzuführen ist.

Der vorliegenden Erfindung lag daher die Aufgabe zugrunde, ein Verfahren zur gezielten photolytischen Abspaltung von Schutzgruppen von auf einem Trägermaterial immobilisierten Nucleosid-Derivaten zu entwickeln, insbesondere von in der DNA-Chip-Herstellung üblichen Schutzgruppen, welches die genannten Nachteile des Standes der Technik nicht aufweist, sondern eine schnelle und vollständige Schutzgruppenabspaltung ermöglicht.

Diese Aufgabe wird erfindungsgemäß dadurch gelöst, daß vor der Photolyse auf das Trägermaterial mit den zu entschützenden Nucleosid-Derivaten eine Schicht eines Gels oder einer viskosen Flüssigkeit aus Polymerverbindungen in Wasser, einem Wasser/C₁-C₄-Alkohol-Gemisch und/oder einem polaren aprotischen Lösemittel aufgetragen wird.

Es hat sich hierbei überraschenderweise gezeigt, daß auf diese Weise thermische und photolytische Nebenreaktionen weitgehend zurückgedrängt werden, so daß die synthetisierten Nucleosid- bzw. Nucleotid-Sequenzen die erforderliche Reinheit aufweisen.

Mit "gezielter photolytischer Abspaltung" ist im Zusammenhang mit dem erfindungsgemäßen Verfahren die selektive Abspaltung photolabiler Schutzgruppen von den geschützten Nucleosid-Derivaten gemeint. Im Rahmen der vorliegenden Erfindung ist es daher neben der vollständigen Abspaltung ebenfalls möglich, nur einen Teil der photolabilen Schutzgruppen abzuspalten, z.B. mit Hilfe von Masken.

Beim Verfahren entsprechend der vorliegenden Erfindung wird auf die Substratoberfläche, d. h. das Trägermaterial mit den immobilisierten Nucleosid-Derivaten bestehend aus Nucleosiden, Nucleotiden oder Oligonucleotiden, eine Schicht eines Gels oder einer viskosen Flüssigkeit aus einer oder mehreren Polymer-Verbindungen in Wasser, einem Wasser/C₁-C₄-Alkohol-Gemisch und/oder einem polaren aprotischen Lösemittel aufgetragen, bevor die Belichtung der Nucleosid-Derivate vorzugsweise von vorne erfolgt. Die Schichtdicke des Gels bzw. der viskosen Flüssigkeit kann hierbei in weiten Grenzen variiert werden, doch hat es sich insbesondere für eine optimale optische Auflösung als vorteilhaft erwiesen, die Schichtdicke auf 0,1 µm bis 5 mm einzustellen, besonders bevorzugt 10 µm bis 5 mm.

Der Anteil an Polymerverbindungen beträgt 1 bis 20 Gew.-%, bezogen auf das Gesamtgewicht des Gels bzw. der viskosen Flüssigkeit Gemäß einer bevorzugten Ausführungsform werden solche Polymere für den Aufbau der Gele herangezogen, die eine Sol/Gel-Übergangstemperatur von 15 bis 90 °C, insbesondere 30 bis 50 °C, besitzen. Der Vorteil dieser Gele liegt darin, daß sie bei Raumtemperatur quasi fest und durch leichtes Erwärmen in den flüssigen Zustand übergeführt werden können, so daß die entsprechenden Gele nach der photolytischen Schutzgruppen-Abspaltung sehr leicht vom jeweiligen Trägermaterial entfernt werden können.

Die eingesetzten Gele sollten vorzugsweise eine Gelstärke von 20 bis 10 000 g/cm², insbesondere 100 bis 1 000 g/cm², aufweisen. Die Gelstärke wird üblicherweise durch Kompressions-Tests bestimmt, die dem Fachmann geläufig sind. Für den Fall der Verwendung von Gelatine kann die Gelstärke auch nach der Bloom-Methode bestimmt werden. Dabei entspricht die Gelstärke der Kraft in Gramm, die von einem definierten zylindrischen Kolben auf der Oberfläche eines 6,67 %igen Gelatine-Gels (erhalten nach 17 Stunden bei 10 °C) aufgebracht werden muß, um eine Eindrucktiefe von 4 mm zu erhalten. Für Gelatine-Gele beträgt die dermaßen bestimmte Gelstärke im erfindungsgemäßen Verfahren dann vorzugsweise 5 bis 300 g.

Im Falle der Verwendung von viskosen Flüssigkeiten sollten diese vorzugsweise eine dynamische Viskosität von 5 bis 40 000 mPa·s, insbesondere 50 bis 15 000 mPa·s, (gemessen bei 25 °C und der jeweiligen Konzentration) aufweisen. Die Art der verwendeten Polymer-Verbindungen ist weitgehend unkritisch, d. h. sie sollen lediglich in Gegenwart von Wasser bzw. dem betreffenden Lösemittel zu den gewünschten Gelen bzw. viskosen Flüssigkeiten führen. Beim erfindungsgemäßen Verfahren können somit eine Reihe von synthetischen oder natürlichen Polymeren eingesetzt werden. Aus der Reihe der synthetischen Polymere haben sich Polyvinylalkohol (PVA), Polyvinylacetal, Polyacrylamid, Polyvinylpyrrolidon (PVP), Polyvinylacetat, Polyethylenimin und Novolake (Polykondensationsprodukte aus Phenol und Formaldehyd) als besonders vorteilhaft erwiesen. Aus der Reihe der natürlichen Polymere werden erfindungsgemäß vorzugsweise Gelatine, Agarose, Agar, Pektin, Galactomannane, Carrageenane, Scleroglucane, Xanthane und Alginate eingesetzt.

Im erfindungsgemäßen Verfahren wird als Lösemittel für das Gel oder die viskose Flüssigkeit Wasser, ein Wasser/C₁-C₄-Alkohol-Gemisch und/oder ein polares aprotisches Lösemittel eingesetzt. Die Alkohole, welche linear oder verzweigt sein können, werden im Gemisch mit Wasser in einem bevorzugten Gewichtsverhältnis von 90/10 bis 10/90 eingesetzt. Die Alkohole können eine oder mehrere OH-Gruppen enthalten und insbesondere ausgewählt werden aus Methanol, Ethanol, 1-Propanol, 2-Propanol, 1-Butanol, 2-Methyl-1-propanol, 2-Butanol, 2-Methyl-2-propanol, Ethylenglykol, 1,3-Propandiol, 1,2-Propandiol, Glycerin, 1,2-Butandiol, 1,3-Butandiol, 1,4-Butandiol und 2,3-Butandiol. Die polaren aprotischen Lösemittel bestehen vorzugsweise aus Dimethylsulfoxid (DMSO), Dimethylformamid (DMF), Dimethylacetamid (DMA), Acetonitril, N-Methylpyrrolidon, Diethylenglykoldimethylether, Tetraethylenglykoldimethylether, Sulfolan, 1,3-Dimethyl-2-imidazolidinon, 1,3-Dimethyltetrahydro-2(1H-)pyrimidinon, 2-Methoxy-1-methylethylacetat oder Propylencarbonat.

Es ist im Rahmen der vorliegenden Erfindung möglich, daß man dem Gel bzw. der viskosen Flüssigkeit noch 0,1 bis 10 Gew.-%, insbesondere 1 bis 5 Gew.-%, an Additiven zusetzt, welche eine rasche und möglichst nebenreaktionsfreie Photolyse ermöglichen. Geeignete Additive sind bspw. Beschleuniger in Form von schwachen Basen wie z. B. Imidazol, Pyrazol, 1-Methylimidazol, 1,8-Diazabicyclo[5,4,0]undec-7-en (DBU), 1,5-Diazabicyclo[4,3,0]non-5-en (DBN), 1,4-Diazabicyclo[2,2,2]octan, Morpholin, N-Methylmorpholin, Piperidin, N-Methylpiperidin, Piperazin, N-Methylpiperazin, Diisopropylethylamin, Triethylamin, Pyridin, Chinolin, Collidin, Lutidin oder Picolin. Desweiteren haben sich Verbindungen wie z. B. Harnstoff, Thioharnstoff, Guanidinhydrochlorid, Glycin, Tris(hydroxy-methyl)-aminomethan, Tris(hydroxymethyl)aminomethanhydrochlorid oder Mannitol als besonders vorteilhaft erwiesen, welche die Photolyse positiv beeinflussen.

Zum Abfangen der bei der Photolyse störenden freien Radikale werden als weitere bevorzugte Additive Redoxpuffer in Form von Histidin, Polyhistidin, Imidazol, Thioharnstoff, Tris-(hydroxymethyl)nitromethan, Natriumazid und/oder Ascorbinsäure eingesetzt.

Zur Beschleunigung der Photolyse können dem Gel bzw. der viskosen Flüssigkeit noch UV-Sensibilisatoren, bspw. in Form von Benzoesäure oder Benzoesäuresalzen (vorzugsweise Alkalisalze wie z. B. Natrium- oder Kaliumsalze), zugesetzt werden. Gemäß einer bevorzugten Ausführungsform werden dem Gel bzw. der viskosen Flüssigkeit noch Konsistenzregler in einer Menge von 0,001 bis 10 Gew.-%, bezogen auf das Gesamtgewicht des Gels bzw. der viskosen Flüssigkeit, auf Basis von Alkalioder Erdalkali-Salzen (wie z. B. NaCl, KCl, CaCl₂) zugesetzt. Es ist im Rahmen der vorliegenden Erfindung jedoch auch möglich, daß die entsprechenden Additive kovalent an die Polymer-Verbindungen gebunden sind und diese in Form von funktionellen Gruppen enthalten sein können. Beispiele für derartige funktionalisierte Polymer-Verbindungen sind bspw. veresterte oder teilveresterte Polyvinylalkohole.

Zur Durchführung des erfindungsgemäßen Verfahrens wird die Nucleosid-Lösung auf ein geeignetes Trägermaterial aufgebracht und immobilisiert. Eine Immobilisierung kann beispielsweise durch Verdunsten des Lösemittels erfolgen. Daneben können die Nucleosid-Derivate durch kovalente Bindung auf der Trägeroberfläche immobilisiert werden. Hierzu wird auf der Substratoberfläche ein Linker kovalent aufgebracht, der terminale OH- oder NH₂-Funktionen aufweist. Diese freien funktionellen Gruppen fungieren direkt als in-situ Startpunkte für die anschließende photolitographische Synthese der Nucleosid-Derivate.

Als besonders vorteilhaft haben sich folgende Nucleosid-Derivate bzw. folgende Schutzgruppen erwiesen: 5'-O-[2-(4-Cyano-2-nitrophenyl)ethoxycarbonyl)thymidin], 5'-O-[2-(2-Chlor-6-nitrophenyl)ethoxycarbonyl])thymidin, 5'-O-[2-(2-Nitrophenyl)-propoxycarbonyl]thymidin (NPPOC-T), 5'-O-[2-(2-Nitrophenyl)propoxythiocarbonyl]thymidin, 5'-O-[2-(4-Brom-2-nitrophenyl)propylsulfonyl]- thymidin, 5'-O-[2-(4-Brom-2-nitrophenyl)propoxycarbonyl]thymidin, 5'-O-[2-(4-Iod-2-nitrophenyl)propoxycarbonyl]thymidin, 5'-O-(α-Methyl-2-nitropiperonyloxycarbonyl)thymidin (MeNPOC-T), 5'-O-[(8-Nitronaphth-1-yl)methoxycarbonyl]thymidin, 5'-O-[1-(3-Nitrothien-2-yl)]ethoxycarbonyl]thymidin (NTEOC-T), 5'-O-[2-(3-Nitrothien-2-yl) propoxycarbonyl]thymidin (NTPOC-T) und 5'-O-[(7-Methoxycumarin-4-yl) methyloxycarbonyl]thymidin (MCMOC-T).

Anschließend wird diese Substratoberfläche mit einer dünnen und homogenen Schicht des Polymergels oder der viskosen Flüssigkeit bedeckt, wobei die Beschichtung der Substratoberfläche vorzugsweise durch Spin-Coating erfolgt.

Danach kann die Photolyse der Nucleosid-Derivate, die im Gel bzw. in der viskosen Flüssigkeit quasi gelöst vorliegen, erfolgen, wobei die Belichtung üblicherweise von vorne erfolgt. Vorzugsweise wird die Photolyse unter Schutzgasatmosphäre, wie z. B. Stickstoff oder Argon, durchgeführt, um mögliche Nebenreaktionen weitgehend zurückzudrängen.

Im Anschluß an die Photolyse wird das Gel bzw. die viskose Flüssigkeit vom Trägermaterial wieder entfernt, was bei Gelen mit einer relativ niedrigen Sol/Gel-Übergangstemperatur rein thermisch oder ansonsten mit geeignetem Lösemittel (DMSO, DMF, Wasser) erfolgen kann.

Die Vorteile der Polymergele bzw. viskosen Lösungen bestehen darin, daß sie ggf. mit den entsprechenden Additiven die Photolyse beschleunigen, Nebenprodukte abfangen, die (Oligo-)Nucleotid-Ketten geeignet ausrichten und die Reaktionswärme abfangen können. Auf diese Weise wird eine schnelle, saubere und vollständige Abspaltung der photolabilen Schutzgruppen von den Nucleosid-Derivaten begünstigt, was wiederum die geforderte Reinheit der synthetisierten Nucleotid- bzw. Oligonucleotid-Sequenzen zur Folge hat.

Die nachfolgenden Beispiele sollen die Erfindung näher veranschaulichen.

Als besonders gut geeignet zur Durchführung des erfindungsgemäßen Verfahrens hat sich die Verwendung einer viskosen Lösung erwiesen, die aus Polyvinylalkohol (mittleres Molekulargewicht 49 000) mit einem Anteil von 4 Gew.-% (Rest Wasser) und 1 Gew.-% Imidazol besteht. Diese Ausführungsform ist in Beispiel 1, Versuch Nr. 18 beschrieben.

### Beispiele

### 1) Allgemeines

Es konnte ein Modell zur Simulation der photolithographischen Festphasensynthese etabliert werden. Hierbei wird eine Nucleosid-Lösung auf dem Boden einer Reaktionskammer einer Mikrotiterplatte aufgebracht. Nach Verdunsten des Lösemittels liegt das photolabil-geschützte Nucleosid auf dem Boden der Reaktionskammer gleichmäßig verteilt vor. Es kann nun "trocken" oder mit einem Gel und/oder einer viskosen Flüssigkeit von Polymerverbindungen überschichtet "von vorne" bestrahlt werden. Die Auswertung der Photolyse erfolgt quantitativ mittels HPLC. Es konnte mit diesem Modell gezeigt werden, daß die Abspaltung bei der trockenen Photolyse deutlich schlechter verläuft als bei Bestrahlung eines Gels bzw. einer viskosen Flüssigkeit von Polymerverbindungen.

### 2) Allgemeine Arbeitsvorschrift für die Belichtung mit Gelen bzw. viskosen Flüssigkeiten von Polymerverbindungen

Auf den Boden eines Mikroreaktionsgefäßes einer Mikrotiterplatte werden 8 µl einer 0,5 mmolaren Lösung (4 µl einer 1 mmolaren Lösung) eines photolabilen Nucleosids in Acetonitril aufgetragen. Nach wenigen Minuten ist das Lösemittel verdunstet. Daraufhin werden idealerweise 30 µl (15 µl bis maximal 100 µl) eines Gels bzw. einer viskosen Flüssigkeit von Polymerverbindungen gleichmäßig aufgetragen (Schichtdicke 3 bis 4 mm). Anschließend wird die Probe bestrahlt (Lichtquelle: Hg-Hochdrucklampe HBO 100 W mit Interferenzfilter Lambda-max 365 nm, Bestrahlungsdauer: 30 Min.). Schließlich wird die bestrahlte Probe mit Methanol/Wasser, Acetonitril/Wasser, Acetonitril/Methanol/Wasser oder ähnlichen geeigneten Lösemitteln verdünnt und in den HPLC-Chromatographen eingespritzt.

### Beispiel 1

### Bestrahlung von 5'-O-[2-(2-Nitrophenyl)-propoxycarbonyl]thymidin (NPPOC-T)

Bestrahlung bei 365 nm, maximale Bestrahlungszeit: 30 min

| Versuch Nr. | Viskose Flüssigkeit bzw. Gel | Ausbeute [%] |
|---|---|---|
| 1 | Ohne viskose Flüssigkeit bzw. Gel | 48 |
| 2 | Gelatine med (1 Gew.-%)⁴⁾ | 51 |
| 3 | Agarose 1¹⁾ (0,5 Gew.-%)⁴⁾ (Gel) | 66 |
| 4 | PVA²⁾ (1 Gew.-%)⁴⁾ | 98 |
| 5 | PVA²⁾ (4 Gew.-%)⁴⁾ | 99 |
| 6 | PVA²⁾ (8 Gew.-%)⁴⁾ | 77 |
| 7 | PVA²⁾ (4 Gew.-%)⁴⁾ + 1 Gew.-% Glycin | 86 |
| 8 | PVA²⁾ (4 Gew.-%)⁴⁾ + 4 Gew.-% Glycin | 86 |
| 9 | PVA²⁾ (4 Gew.-%)⁴⁾ + 1 Gew.-% Tris | 97 |
| 10 | PVA²⁾ (4 Gew.-%)⁴⁾ + 1 Gew.-% TrisHCl | 74 |
| 11 | PVA²⁾ (4 Gew.-%)⁴⁾ + 1 Gew.-% Trisnitro | 49 |
| 12 | PVA²⁾ (4 Gew.-%)⁴⁾ + 1 Gew.-% PVP³⁾ | 56 |
| 13 | PVA²⁾ (4 Gew.-%)⁴⁾ + 1 Gew.-% Guhy | 93 |
| 14 | PVA²⁾ (4 Gew.-%)⁴⁾ + 1 Gew.-% Harnstoff | 96 |
| 15 | PVA²⁾ (4 Gew.-%)⁴⁾ + 1 Gew.-% Thioharnstoff | 81 |
| 16 | PVA²⁾ (4 Gew.-%)⁴⁾ + 1 Gew.-% Mannitol | 96 |
| 17 | PVA²⁾ (4 Gew.-%)⁴⁾ + 1 Gew.-% Histidin | 99 |
| 18 | PVA²⁾ (4 Gew.-%)⁴⁾ + 1 Gew.-% Imidazol | 100 |

| | | |
|---|---|---|
| ¹⁾ Agarose I der Fa. Amresco, Solon, Ohio (USA) | | |
| ²⁾ mittleres Molekulargewicht 49 000 | | |
| ³⁾ Polyvinylpyrrolidon K25 der Fa. Fluka, Buchs (Schweiz) ⁴⁾ Rest Wasser | | |

### Beispiel 2

### Bestrahlung von 5'-O-(α-Methyl-2-nitropiperonyloxycarbonyl)thymidin (MeNPOC-T)

Bestrahlung bei 365 nm, maximale Bestrahlungszeit: 30 min

| Versuch Nr. | Viskose Flüssigkeit | Ausbeute [%] |
|---|---|---|
| 1 | Ohne viskose Flüssigkeit | 48 |
| 2 | PVA¹⁾ (4 Gew.-%)³⁾ | 78 |
| 3 | PVA¹⁾ (8 Gew.-%)³⁾ | 83 |
| 4 | PVA¹⁾ (4 Gew.-%)³⁾ + 1 Gew.-% Glycin | 79 |
| 5 | PVA¹⁾ (4 Gew.-%)³⁾ + 4 Gew.-% Glycin | 79 |
| 6 | PVA¹⁾ (4 Gew.-%)³⁾ + 1 Gew.-% TRIS | 78 |
| 7 | PVA¹⁾ (4 Gew.-%)³⁾ + 1 Gew.-% TRIS-HCl | 83 |
| 8 | PVA¹⁾ (4 Gew.-%)³⁾ + 1 Gew.-% TRIS-nitro | 78 |
| 9 | PVA¹⁾ (4 Gew.-%)³⁾ + 4 Gew.-% TRIS-nitro | 79 |
| 10 | PVA¹⁾ (4 Gew.-%)³⁾ + 1 Gew.-% PVP²⁾ | 82 |
| 11 | PVA¹⁾ (4 Gew.-%)³⁾ + 4 Gew.-% PVP²⁾ | 79 |
| 12 | PVA¹⁾ (4 Gew.-%)³⁾ + 1 Gew.-% Guhy | 81 |
| 13 | PVA¹⁾ (4 Gew.-%)³⁾ + 1 Gew.-% Harnstoff | 80 |
| 14 | PVA¹⁾ (4 Gew.-%)³⁾ + 1 Gew.-% Thioharnstoff | 76 |

| | | |
|---|---|---|
| ¹⁾ mittleres Molekulargewicht 49 000 | | |
| ²⁾ Polyvinylpyrrolidon K25 der Fa. Fluka, Buchs (Schweiz) ³⁾ Rest Wasser | | |

### Beispiel 3

### Bestrahlung von 5'-O-[2-(2-Nitrophenyl)-propoxycarbonyl]thymidin (NPPOC-T)

Bestrahlung bei 365 nm, maximale Bestrahlungszeit: 30 min

| Versuch Nr. | Viskose Flüssigkeit | Ausbeute [%] |
|---|---|---|
| 1 | Ohne viskose Flüssigkeit | 48 |
| 2 | PVA¹⁾ (4 Gew.-%), DMSO | 66 |
| 3 | PVA¹⁾ (4 Gew.-%) + 1 Gew.-% Imidazol, DMSO | 73 |
| 4 | PVA¹⁾ (10 Gew.-%) + 1 Gew.-% Imidazol, DMSO | 98 |
| 5 | PVA¹⁾ (4 Gew.-%) + 1 Gew.-% 1-Methylimidazol, DMSO | 96 |
| 6 | PVA¹⁾ (8 Gew.-%)+ 1 Gew.-% 1-Methylimidazol, DMSO | 95 |
| 7 | PVA¹⁾ (10 Gew.-%) + 1 Gew.-% 1-Methylimidazol, DMSO | 88 |
| 8 | PVA¹⁾ (15 Gew.-%) + 1 Gew.-% 1-Methylimidazol, DMSO | 88 |
| 9 | PVA¹⁾ (4 Gew.-%) + 1 Gew.-% 1-Methylimidazol, DMA | 97 |
| 10 | PVA¹⁾ (10 Gew.-%) + 1 Gew.-% 1-Methylimidazol, DMA | 75 |
| 11 | PVAcetat²⁾ (4 Gew.-%), DMSO | 91 |
| 12 | PVAcetat²⁾ (4 Gew.-%) + 1 Gew.-% 1-Methylimidazol, DMSO | 81 |

| | | |
|---|---|---|
| ¹⁾ mittleres Molekulargewicht 49 000 | | |
| ²⁾ mittleres Molekulargewicht 170 000 | | |

### Beispiel 4

### Bestrahlung von 2'-Desoxy-5'-O-[2-(2-nitrophenyl)propoxycarbonyl]-N⁴phenoxyacetylcytidin (NPPOC-dC^{PAC})

Bestrahlung bei 365 nm, maximale Bestrahlungszeit: 30 min

| Versuch Nr. | Viskose Flüssigkeit | Ausbeute [%] |
|---|---|---|
| 1 | Ohne viskose Flüssigkeit | 35 |
| 2 | PVA¹⁾ (4 Gew.-%) + 1 Gew.-% 1-Methylimidazol, DMSO | 100 |

| | | |
|---|---|---|
| ¹⁾ mittleres Molekulargewicht 49 000 | | |

### Beispiel 5

### Bestrahlung von 2'-Desoxy-5'-O-[2-(2-nitrophenyl)propoxycarbonyl]-N⁶phenoxyacetyladenosin (NPPOC-dA^{PAC})

Bestrahlung bei 365 nm, maximale Bestrahlungszeit: 30 min

| Versuch Nr. | Viskose Flüssigkeit | Ausbeute [%] |
|---|---|---|
| 1 | Ohne viskose Flüssigkeit | 44 |
| 2 | PVA¹⁾ (4 Gew.-%) + 1 Gew.-% 1-Methylimidazol, DMSO | 95 |
| 3 | PVA¹⁾ (10 Gew.-%) + 1 Gew.-% 1-Methylimidazol, DMSO | 91 |
| 4 | PVAcetat²⁾ (4 Gew.-%) + 1 Gew.-% 1-Methylimidazol, DMSO | 90 |

| | | |
|---|---|---|
| ¹⁾ mittleres Molekulargewicht 49 000 | | |
| ²⁾ mittleres Molekulargewicht 170 000 | | |

### Beispiel 6

### Bestrahlung von 2'-Desoxy-5'-O-[2-(2-nitrophenyl)propoxycarbonyl]-N²phenoxyacetylguanosin (NPPOC-dG^{PAC})

Bestrahlung bei 365 nm, maximale Bestrahlungszeit: 30 min

| Versuch Nr. | Viskose Flüssigkeit | Ausbeute [%] |
|---|---|---|
| 1 | Ohne viskose Flüssigkeit | 36 |
| 2 | PVA¹⁾ (4 Gew.-%) + 1 Gew.-% 1-Methylimidazol, DMSO | 93 |

| | | |
|---|---|---|
| ¹⁾ mittleres Molekulargewicht 49 000 | | |

### Beispiel 7

### Bestrahlung von 5'-O-(α-Methyl-2-nitropiperonyloxycarbonyl)thymidin (MeNPOC-T)

Bestrahlung bei 365 nm, maximale Bestrahlungszeit: 30 min

| Versuch Nr. | Viskose Flüssigkeit | Ausbeute [%] |
|---|---|---|
| 1 | Ohne viskose Flüssigkeit | 48 |
| 2 | PVA¹⁾ (4 Gew.-%) + 1 Gew.-% 1-Methylimidazol, DMSO | 74 |
| 3 | PVA¹⁾ (10 Gew.-%) + 1 Gew.-% 1-Methylimidazol, DMSO | 72 |

| | | |
|---|---|---|
| ¹⁾ mittleres Molekulargewicht 49 000 | | |

## Patentansprüche

1. Verfahren zur gezielten photolytischen Abspaltung von Schutzgruppen von auf einem Trägermaterial immobilisierten Nucleosid-Derivaten, **dadurch gekennzeichnet, daß** es die folgenden Schritte umfasst:
zuerst Aufbringen einer Schicht aus einem Gel oder einer viskosen Flüssigkeit auf den auf dem Trägermaterial immobilisierten Nucleosid-Derivaten, wobei das Gel oder die viskose Flüssigkeit eine oder mehrere Polymerverbindungen mit einem Gehalt von 1 bis 20 Gew.-%, bezogen auf das Gesamtgewicht des Gels oder der viskosen Flüssigkeit, und wenigstens einen Vertreter aus Wasser, Wasser/C₁-C₄-Alkohol-Gemischen und polaren aprotischen Lösemitteln umfasst, und
Belichten der Nucleosid-Derivate zur photolytischen Abspaltung der Schutzgruppen.

2. Verfahren nach Anspruch 1, **dadurch gekennzeichnet, daß** die Schichtdicke des Gels oder der viskosen Flüssigkeit 0,1 µm bis 5 mm beträgt.

3. Verfahren nach Anspruch 1 oder 2, **dadurch gekennzeichnet, daß** das Gel eine Sol/Gel-Übergangstemperatur von 15 bis 90 °C, insbesondere 30 bis 50 °C, besitzt.

4. Verfahren nach einem der Ansprüche 1 bis 3, **dadurch gekennzeichnet, daß** das Gel eine Gelstärke von 20 bis 10 000 g/cm², insbesondere 100 bis 1 000 g/cm², besitzt.

5. Verfahren nach Anspruch 1 oder 2, **dadurch gekennzeichnet, daß** die viskose Flüssigkeit eine dynamische Viskosität von 5 bis 40 000 mPa·s, insbesondere 50 bis 15 000 mPa·s, bei 25 °C und der jeweiligen Konzentration besitzt.

6. Verfahren nach einem der Ansprüche 1 bis 5, **dadurch gekennzeichnet, daß** die eine oder mehreren Polymerverbindungen ausgewählt werden aus Polyvinylalkohol, Polyvinylacetal, Polyacrylamid, Polyvinylpyrrolidon, Polyvinylacetat, Polyethylenimin, Novolaken, Gelatine, Agarose, Agar, Pektin, Galactomannanen, Carrageenanen, Scleroglucanen, Xanthanen und Alginaten.

7. Verfahren nach einem der Ansprüche 1 bis 6, **dadurch gekennzeichnet, daß** das Wasser/C₁-C₄-Alkohol-Gemisch im Gewichtsverhältnis von 90/10 bis 10/90 eingesetzt wird.

8. Verfahren nach einem der Ansprüche 1 bis 7, **dadurch gekennzeichnet, daß** das polare aprotische Lösemittel ausgewählt wird aus Dimethylsulfoxid (DMSO), Dimethylformamid (DMF), Dimethylacetamid (DMA), Acetonitril, N-Methylpyrrolidon, Diethylenglykoldimethylether, Tetraethylenglykoldimethylether, Sulfolan, 1,3-Dimethyl-2-imidazolidinon, 1,3-Dimethyltetrahydro-2(1H-)pyrimidinon, 2-Methoxy-1-methylethylacetat und Propylencarbonat.

9. Verfahren nach einem der Ansprüche 1 bis 8, **dadurch gekennzeichnet, daß** das Gel oder die viskose Flüssigkeit zusätzlich ein oder mehrere Additive in einer Menge von 0,1 bis 10 Gew.-%, insbesondere 1 bis 5 Gew.-%, umfasst, ausgewählt aus Beschleunigern, Redoxpuffern und UV-Sensibilisatoren.

10. Verfahren nach Anspruch 9, **dadurch gekennzeichnet, daß** die Beschleuniger ausgewählt werden aus Imidazol, Pyrazol, 1-Methylimidazol, Harnstoff, Thioharnstoff, Guanidinhydrochlorid, Glycin, Tris(hydroxymethyl)aminomethan, Tris(hydroxymethyl)aminomethan-hydrochlorid und Mannitol.

11. Verfahren nach Anspruch 9, **dadurch gekennzeichnet, daß** die Redoxpuffer ausgewählt werden aus Histidin, Polyhistidin, Imidazol, Thioharnstoff, Tris(hydroxymethyl)nitromethan, Natriumazid und Ascorbinsäure.

12. Verfahren nach Anspruch 9, **dadurch gekennzeichnet, daß** die UV-Sensibilisatoren ausgewählt werden aus Benzoesäure und Benzoesäuresalzen.

13. Verfahren nach einem der Ansprüche 9 bis 12, **dadurch gekennzeichnet, daß** alle oder ein Teil der Additive kovalent an die Polymerverbindungen gebunden sind.

14. Verfahren nach einem der Ansprüche 1 bis 13, **dadurch gekennzeichnet, daß** das Gel oder die viskose Flüssigkeit zusätzlich einen oder mehrere Konsistenzregler, ausgewählt aus Alkali- oder Erdalkali-Salzen, in einer Menge von 0,001 bis 10 Gew.-% umfaßt, bezogen auf das Gesamtgewicht des Gels oder der viskosen Flüssigkeit.

15. Verfahren nach einem der Ansprüche 1 bis 14, **dadurch gekennzeichnet, daß** das Gel oder die viskose Flüssigkeit durch Spin-Coating auf die Nucleosid-Derivate auf dem Trägermaterial aufgebracht wird.

16. Verfahren nach einem der Ansprüche 1 bis 15, **dadurch gekennzeichnet, daß** die Photolyse unter Schutzgasatmosphäre, insbesondere Stickstoff oder Argon, durchgeführt wird.

17. Verfahren nach einem der Ansprüche 1 bis 16, **dadurch gekennzeichnet, daß** die Belichtung von der Seite des Trägermaterials mit den darauf immobilisierten Nucleosid-Derivaten erfolgt.

18. Verfahren nach einem der Ansprüche 1 bis 4 und 6 bis 17, **dadurch gekennzeichnet, daß** das Gel nach der photolytischen Schutzgruppen-Abspaltung durch Erwärmen in den flüssigen Zustand überführt und vom Trägermaterial entfernt wird.

## Claims

1. Process for specific photolytic cleavage of protective groups from nucleoside derivatives immobilised on a support material, **characterised in that** it comprises the following steps:
first of all applying a layer of a gel or a viscous liquid to the nucleoside derivatives immobilised on the support material, wherein the gel or the viscous liquid comprises one or more polymer compounds containing 1 to 20 wt.%, based on the total weight of the gel or the viscous liquid, and at least one representative from water, water/C₁-C₄ alcohol mixtures and polar aprotic solvents, and
illuminating the nucleoside derivatives for photolytic cleavage of the protective groups.

2. Process according to claim 1, **characterised in that** the layer thickness of the gel or of the viscous liquid is 0.1 µm to 5 mm.

3. Process according to claim 1 or 2, **characterised in that** the gel has a sol/gel transition temperature of 15 to 90°C, in particular 30 to 50°C.

4. Process according to one of claims 1 to 3, **characterised in that** the gel has a gel thickness of 20 to 10,000 g/cm², in particular 100 to 1,000 g/cm².

5. Process according to claim 1 or 2, **characterised in that** the viscous liquid has a dynamic viscosity of 5 to 40,000 mPa.s, in particular 50 to 15,000 mPa.s, at 25°C and the particular concentration.

6. Process according to one of claims 1 to 5, **characterised in that** the one or more polymer compounds are selected from polyvinyl alcohol, polyvinyl acetal, polyacrylamide, polyvinylpyrrolidone, polyvinyl acetate, polyethyleneimine, novolaks, gelatine, agarose, agar, pectin, galactomannans, carrageenans, scleroglucans, xanthenes and alginates.

7. Process according to one of claims 1 to 6, **characterised in that** the water/C₁-C₄ alcohol mixture is used in a weight ratio of 90/10 to 10/90.

8. Process according to one of claims 1 to 7, **characterised in that** the polar aprotic solvent is selected from dimethylsulphoxide (DMSO), dimethylformamide (DMF), dimethylacetamide (DMA), acetonitrile, N-methylpyrrolidone, diethylene glycol dimethylether, tetraethylene glycol dimethylether, sulpholane, 1,3-dimethyl-2-imidazolidinone, 1,3-dimethyltetrahydro-2(1H)pyrimidinone, 2-methoxy-1-methylethyl acetate and propylene carbonate.

9. Process according to one of claims 1 to 8, **characterised in that** the gel or the viscous liquid additionally comprises one or more additives in a quantity of 0.1 to 10 wt.%, in particular 1 to 5 wt.%, selected from accelerators, redox buffers and UV sensitisers.

10. Process according to claim 9, **characterised in that** the accelerators are selected from imidazole, pyrazole, 1-methylimidazole, urea, thiourea, guanidine hydrochloride, glycin, tris(hydroxymethyl)aminomethane, tris(hydroxymethyl)aminomethane hydrochloride and mannitol.

11. Process according to claim 9, **characterised in that** the redox buffers are selected from histidine, polyhistidine, imidazole, thiourea, tris(hydroxymethyl)nitromethane, sodium azide and ascorbic acid.

12. Process according to claim 9, **characterised in that** the UV sensitisers are selected from benzoic acid and benzoic acid salts.

13. Process according to one of claims 9 to 12, **characterised in that** all or some of the additives are bound covalently to the polymer compounds.

14. Process according to one of claims 1 to 13, **characterised in that** the gel or the viscose liquid additionally comprises one or more consistency regulators, selected from alkali metal or alkaline earth metal salts, in a quantity of 0.001 to 10 wt.%, based on the total weight of the gel or the viscous liquid.

15. Process according to one of claims 1 to 14, **characterised in that** the gel or the viscous liquid is applied to the nucleoside derivatives on the support material by spin-coating.

16. Process according to one of claims 1 to 15, **characterised in that** the photolysis is carried out under protective gas atmosphere, in particular nitrogen or argon.

17. Process according to one of claims 1 to 16, **characterised in that** illumination is effected from the side of the support material with the nucleoside derivatives immobilised thereon.

18. Process according to one of claims 1 to 4 and 6 to 17, **characterised in that** the gel is converted to the liquid state by heating after the photolytic cleavage of protective groups and removed from the support material.

## Revendications

1. Procédé de séparation photolytique à dessein de groupes protecteurs, de dérivés de nucléosides immobilisés sur un matériau support, **caractérisé en ce qu'**il comprend les étapes suivantes :
d'abord application d'une couche formée d'un gel ou d'un liquide visqueux, sur les dérivés de nucléosides, immobilisés sur le matériau support, le gel ou le liquide visqueux comprenant une ou plusieurs combinaisons polymères, ayant une teneur de 1 à 20% en poids en se référant au poids total du gel ou du liquide visqueux, et au moins un représentant du groupe formé de l'eau, des mélanges eau/alcool en C₁-C₄ et de solvants polaires aprotiques, et éclairement des dérivés nucléosides pour obtenir la séparation photolytique des groupes protecteurs.

2. Procédé selon la revendication 1, **caractérisé en ce que** l'épaisseur de couche du gel ou du liquide visqueux est de 0,1 µm à 5 mm.

3. Procédé selon la revendications 1 ou 2, **caractérisé en ce que** le gel présente une température de transition sol/gel de 15 à 90°C, en particulier de 30 à 50°C.

4. Procédé selon l'une des revendications 1 à 3, **caractérisé en ce que** le gel présente une force de gel de 20 à 10.000 g/cm², en particulier de 100 à 1.000 g/cm².

5. Procédé selon la revendication 1 ou 2, **caractérisé en ce que** le liquide visqueux présente une viscosité dynamique de 5 à 40.000 mPa·s, en particulier 50 à 15.000 mPa·s, à 25°C et à la concentration respective.

6. Procédé selon l'une des revendications 1 à 5, **caractérisé en ce que** les une ou plusieurs combinaisons polymères sont sélectionnées parmi le groupe formé d'alcool polyvinylique, d'acétal de polyvinyle, de polyacrylamide, de polyvinyl pyrrolidon, d'acétate de polyvinyle, de polyéthylènimine, de novolaques, de gélatines, d'agarose, d'agar, de pectine, de galactomannanes, de carrageenanes, de scléroglucanes, de xanthanes et alginates.

7. Procédé selon l'une des revendications 1 à 6, **caractérisé en ce que** le mélange eau/alcool en C₁ à C₄ est utilisé en un rapport de poids de 90/10 à 10/90.

8. Procédé selon l'une des revendications 1 à 7, **caractérisé en ce que** le solvant aprotique polaire est choisi parmi le diméthyl sulfoxide (DMSO), diméthyl formamide (DMF), diméthyl acétamide (DMA), acétonitrile, N-méthyle pyrrolidone, diéthylène glycoldiméthyléther, tétraéthylène glycoldiméthyléther, sulfolane, 1,3-diméthyl-2-imidazolidinone, 1,3-diméthyl tétrahydro-2-(1H-)pyrimidinone, 2-méthoxy-1-méthyl éthyl acétate et propylène carbonate.

9. Procédé selon l'une des revendications 1 à 8, **caractérisé en ce que** le gel ou liquide visqueux comprend en plus un ou plusieurs additifs en une quantité de 0,1 à 10% en poids, en particulier de 1 à 5 % en poids, sélectionnés parmi les accélérateurs, les tampons redox et les sensibilisateurs aux UV.

10. Procédé selon la revendication 9, **caractérisé en ce que** les accélérateurs sont choisis parmi imidazole, pyrazole, 1-méthyl imidazole, urée, thio-urée, hydrochlorure de guanidine, glycine, tris(hydroxyméthyl)aminométhane, tris(hydroxyméthyl)aminométhane hydrochlorure et mannitol.

11. Procédé selon la revendication 9, **caractérisé en ce que** les tampons redox sont choisis parmi histidine, polyhistidine, imidazole, thio-urée, tris(hydroxy méthyle)nitrométhane, azide de sodium et acide ascorbique.

12. Procédé selon la revendication 9, **caractérisé en ce que** les sensibilisateurs aux UV sont choisis parmi l'acide benzoïque et les sels d'acide benzoïques.

13. Procédé selon l'une des revendications 9 à 12, **caractérisé en ce que** tout les additifs ou une partie des additifs sont liés de façon covalente aux combinaisons polymères.

14. Procédé selon l'une des revendications 1 à 13, **caractérisé en ce que** le gel ou liquide visqueux comprend en plus un ou plusieurs régulateurs de consistance, choisi(s) parmi des sels alcalins ou alcalino-terreux, en une quantité de 0,001 à 10% en poids, en se référant au poids total du gel ou du liquide visqueux.

15. Procédé selon l'une des revendications 1 à 14, **caractérisé en ce que** le gel ou le liquide visqueux est appliqué sur les dérivés de nucléoside, sur le matériau support, par un revêtement par centrifugation.

16. Procédé selon l'une des revendications 1 à 15, **caractérisé en ce que** la photolyse est conduite sous une atmosphère de gaz protecteur, en particulier de l'azote ou de l'argon.

17. Procédé selon l'une des revendications 1 à 16, **caractérisé en ce que** l'éclairement se fait depuis le côté du matériau support comportant les dérivés de nucléoside, ayant été immobilisés sur lui.

18. Procédé selon l'une des revendications 1 à 4 et 6 à 17, **caractérisé en ce que** le gel est passé à l'état liquide par chauffage, après séparation photolytique des groupes protecteurs, et est enlevé du matériau support.
